# EUROPEAN PATENT APPLICATION

(11) **EP 4 699 603 A1**
(43) Date of publication of application: **25.02.2026**
(21) Application number: 25197357.4
(22) Date of filing: 21.08.2025
(51) Int. Cl.: A61K 9/48, A61K 9/16, A61K 31/445

(54) **CAPSULE FORMULATIONS COMPRISING MIGALASTAT**

(30) Priority: 23.08.2024 TR 2024011149
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); GULER, Tolga, Istanbul (TR); GOKSEN ERSOY, Elif, Istanbul (TR); OZTAS, Necla, Istanbul (TR)
(74) Representative: Genç Ilhan, Oznur

(57) **Abstract**

The present invention relates to a capsule formulation comprising migalastat hydrochloride wherein migalastat hydrochloride having a d (0.9) particle size less than 200 µm. The present invention also relates to a simple, rapid, cost effective, time-saving and industrially convenient process of preparing the capsule.

## Description

### Field of Invention

The present invention relates to a capsule formulation comprising migalastat hydrochloride wherein migalastat hydrochloride having a d (0.9) particle size less than 200 µm. The present invention also relates to a simple, rapid, cost effective, time-saving and industrially convenient process of preparing the capsule.

### Background of the Invention

Fabry disease is a multi-systemic, X-linked lysosomal storage disease caused by decreased activity of alpha-galactosidase A and results in lysosomal accumulations of neutral glycosphingolipids and globotriaosylceramide GL-3. Angiokeratoma corporis diffusum is the typical skin lesion seen in Fabry disease and is linked to renal involvement, especially proteinuria.

Migalastat was developed by Amicus therapeutics and granted approval in 2018 to treat fabry disease, a rare and progressive lysosomal storage disorder caused by mutations in the galactosidase alpha gene.

Migalastat, also known as (2R,3S,4R,5S) - 2- (hydroxymethyl) piperidine-3,4,5-triol, having the following Formula (I):

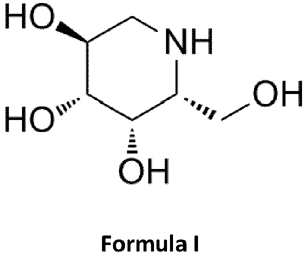

Migalastat is an alpha-galactosidase A chaperone used for the treatment of Fabry disease in patients with an amenable galactosidase alpha gene (GLA) variant.

Migalastat is approved and sold under the brand name Galafold^{®}. It is subsequently an oral pharmacological chaperone of alpha-Gal A for the treatment of Fabry disease in adults who have amenable GLA variants. In these patients, migalastat works by stabilizing the body's dysfunctional alpha-Gal A enzyme so that it can clear the accumulation of glycosphingolipid disease substrate.

Migalastat hydrochloride is a white to almost white crystalline solid. It is freely soluble between pH 1.2 and pH 7.5 in aqueous media.

WO2019046244 (A1) discloses the use of migalastat for the treatment of Fabry disease.

The patent EP3698792A1 discloses a pharmaceutical composition comprising migalastat hydrochloride, magnesium stearate and pregelatinized starch for the treatment of Fabry disease.

There may be some problems in the formulation due to the use of high amounts of active ingredients. When we look at the previous technique, we see that no studies have been conducted on this subject.

There is thus still a need for a capsule formulation comprises migalastat hydrochloride that provides a tablet having the desired dissolution profile and content uniformity, flowability, high stability, and provides additional advantages over the prior art.

### Detailed description of the Invention

The main object of the present invention is a capsule formulation comprising migalastat hydrochloride which has the desired dissolution profile and content uniformity, flowability.

Another object of the present invention is to provide a capsule formulation comprising migalastat hydrochloride which has high stability.

Another object of the present invention is to provide a preparation process of a capsule formulation comprising migalastat hydrochloride which is a simple and cost-effective process.

Migalastat hydrochloride is used in high amounts (approximately 65% above) in the formulation. Therefore, ensuring fluidity and homogeneity in the formulation is of extra importance. In our studies, we have seen that the particle size of migalastat hydrochloride is very important in ensuring homogeneity and powder flowability and therefore in ensuring the dissolution profile. The use of migalastat hydrochloride in the particle sizes listed below helped to provide the desired dissolution profile.

As used herein, 'particle size' means the cumulative volume size distribution as tested by any conventionally accepted method such as the laser diffraction method (i.e. Malvern analysis). The term d (0.9) means the size at which %90 by volume of the particles are finer, the term d (0.5) means the size at which %50 by volume of the particles are finer, the term d (0.1) means the size at which %10 by volume of the particles are finer.

According to main embodiment of the invention, a capsule formulation comprises migalastat hydrochloride wherein migalastat hydrochloride having a d (0.9) particle size less than 200 µm.

According to one embodiment, migalastat hydrochloride has a d (0.9) particle size between 1 µm and 24 µm or between 25 µm and 53 µm or between 55 µm and 87 µm or between 90 µm and 108 µm or between 110 µm and 147 µm or between 150 µm and 183 µm or between 185 µm and 200 µm.

According to an embodiment of the present invention, the amount of migalastat hydrochloride is between 65.0% and 78.0% by weight of the total formulation. Preferably, the amount of migalastat hydrochloride is between 70.0% and 73.0% by weight of the total formulation.

According to an embodiment of the present invention, the capsule formulation further comprises at least one pharmaceutically acceptable excipient which is selected from the group comprising diluents or lubricants or a mixture thereof.

Suitable diluents are selected from the group comprising pregelatinized starch, microcrystalline cellulose, corn starch, lactose monohydrate, lactose, dibasic calcium phosphate, mannitol, spray-dried mannitol, dextrose, sucrose, fructose, maltose, sorbitol, xylitol, inositol, kaolin, inorganic salts, calcium salts, polysaccharides, dicalcium phosphate, sodium chloride, maltodextrin, sucrose-maltodextrin mixture, trehalose, sodium carbonate, sodium bicarbonate, calcium carbonate or a mixture thereof.

According to this embodiment of the present invention, the amount of diluent is between 20.0% and 35.0% by weight of the total formulation. Preferably, the amount of diluent is between 25.0% and 30.0% by weight of the total formulation.

According to an embodiment of the present invention, the diluent is pregelatinized starch or corn starch or mixtures thereof.

Suitable lubricants are selected from the group comprising sodium stearyl fumarate, fatty acid, stearic acid, magnesium stearate, calcium stearate, sodium stearate, stearic acid, glyceryl monostearate, hydrogenated oils, polyethylene glycol, fatty alcohol, fatty acid ester, glyceryl behenate, mineral oil, sodium benzoate or a mixture thereof.

According to an embodiment of the present invention, the lubricant is sodium stearyl fumarate or stearic acid. Thanks to these lubricants, stability is also ensured along with homogeneous powder.

According to this embodiment of the present invention, the amount of lubricant is between 0.1% and 3.0% by weight of the total formulation.

According to this embodiment of the present invention, the capsule formulation comprises;
- Migalastat hydrochloride having a d (0.9) particle size less than 200 µm
- Pregelatinized starch or corn starch
- Sodium stearyl fumarate

According to this embodiment of the present invention, the capsule formulation comprises;
- Migalastat hydrochloride having a d (0.9) particle size less than 200 µm
- Pregelatinized starch or Corn starch
- Stearic acid

### Example 1:

| **Ingredients** | **Amount (%)** | **Amount (%)** |
|---|---|---|
| Migalastat HCl | 65-78 | 72.5 |
| Pregelatinized starch | 20-35 | 26.5 |
| Sodium stearyl fumarate | 0.1-3 | 1 |
| **Total** | **100** | **100** |

a) Mixing migalastat HCl and pregelatinized starch,
b) Compacting the powder mixture and obtained dry granules,
c) Adding sodium stearyl fumarate and then mixing,
d) Filling the mixture into capsules.

### Example 2:

| **Ingredients** | **Amount (%)** | **Amount (%)** |
|---|---|---|
| Migalastat HCl | 65-78 | 72.5 |
| Pregelatinized starch | 20-35 | 26.5 |
| Stearic acid | 0.1-3 | 1 |
| **Total** | **100.00** | **100.00** |

a) Mixing migalastat HCl and pregelatinized starch,
b) Compacting the powder mixture and obtained dry granules,
c) Adding stearic acid and then mixing,
d) Filling the mixture into capsules.

### Example 3:

| **Ingredients** | **Amount (%)** | **Amount (%)** |
|---|---|---|
| Migalastat HCl | 65-78 | 72.5 |
| Corn starch | 20-35 | 26.5 |
| Stearic acid | 0.1-3 | 1 |
| **Total** | **100.00** | **100.00** |

a) Mixing migalastat HCl and corn starch,
b) Compacting the powder mixture and obtained dry granules,
c) Adding stearic acid and then mixing,
d) Filling the mixture into capsules.

## Claims

1. A capsule formulation comprising migalastat hydrochloride wherein migalastat hydrochloride having a d (0.9) particle size less than 200 µm.

2. The capsule formulation according to claim 1, wherein migalastat hydrochloride has a d (0.9) particle size between 1 µm and 24 µm or between 25 µm and 53 µm or between 55 µm and 87 µm or between 90 µm and 108 µm or between 110 µm and 147 µm or between 150 µm and 183 µm or between 185 µm and 200 µm.

3. The capsule formulation according to claim 1, wherein the amount of migalastat hydrochloride is between 65.0% and 78.0%, preferably between 70.0% and 73.0% by weight of the total formulation.

4. The capsule formulation according to any preceding claim, wherein the capsule formulation further comprises at least one pharmaceutically acceptable excipient which is selected from the group comprising diluents or lubricants or a mixture thereof.

5. The capsule formulation according to claim 4, wherein diluents are selected from the group comprising pregelatinized starch, microcrystalline cellulose, corn starch, lactose monohydrate, lactose, dibasic calcium phosphate, mannitol, spray-dried mannitol, dextrose, sucrose, fructose, maltose, sorbitol, xylitol, inositol, kaolin, inorganic salts, calcium salts, polysaccharides, dicalcium phosphate, sodium chloride, maltodextrin, sucrose-maltodextrin mixture, trehalose, sodium carbonate, sodium bicarbonate, calcium carbonate or a mixture thereof.

6. The capsule formulation according to claim 5, wherein the diluent is pregelatinized starch.

7. The capsule formulation according to claim 5 or 6, wherein, the amount of diluent is between 20.0% and 35.0% by weight of the total formulation.

8. The capsule formulation according to claim 4, wherein lubricants are selected from the group comprising sodium stearyl fumarate, fatty acid, stearic acid, magnesium stearate, calcium stearate, sodium stearate, stearic acid, glyceryl monostearate, hydrogenated oils, polyethylene glycol, fatty alcohol, fatty acid ester, glyceryl behenate, mineral oil, sodium benzoate or a mixture thereof.

9. The capsule formulation according to claim 8, wherein the lubricant is sodium stearyl fumarate or stearic acid.

10. The capsule formulation according to claim 8 or 9, wherein the amount of lubricant is between 0.1% and 3.0% by weight of the total formulation.

11. The capsule formulation according to claim 4, wherein the formulation comprising;
• Migalastat hydrochloride having a d (0.9) particle size less than 200 µm
• Pregelatinized starch
• Sodium stearyl fumarate

12. The capsule formulation according to claim 4, wherein the capsule formulation comprising;
• Migalastat hydrochloride having a d (0.9) particle size less than 200 µm
• Pregelatinized starch
• Stearic acid
